# EUROPEAN PATENT SPECIFICATION

(11) **EP 2 371 359 B1**
(45) Date of publication and mention of the grant of the patent: **03.08.2016**
(21) Application number: 11157779.7
(22) Date of filing: 11.03.2011
(51) Int. Cl.: A61K 9/70, A61K 47/02, A61K 31/137

(54) **Selegiline-containing adhesive preparation**
Selegilinhaltige Haftmittelherstellung
Préparation adhésive contenant de la sélégiline

(30) Priority: 12.03.2010 JP 2010056629
(43) Date of publication of application: 05.10.2011
(73) Proprietor: Fujimoto Co., Ltd., Matsubara-shi Osaka (JP)
(72) Inventor: Nishiura, Eri, Ibaraki-shi Osaka (JP); Ameyama, Satoshi, Ibaraki-shi Osaka (JP); Nakamura, Koji, Ibaraki-shi Osaka (JP); Inosaka, Keigo, Ibaraki-shi Osaka (JP); Hori, Mitsuhiko, Ibaraki-shi Osaka (JP)
(74) Representative: Grünecker Patent- und Rechtsanwälte PartG mbB

(56) References cited:
- EP-A1- 0 904 778
- EP-A1- 1 731 143
- EP-A2- 1 044 684
- EP-A2- 2 364 733
- EP-A2- 2 371 360
- WO-A1-00/59475
- WO-A1-96/12472

## Description

### FIELD OF THE INVENTION

This invention relates to an adhesive preparation which comprises a pharmaceutically acceptable salt of (-)-(R)-N,α-dimethyl-N-2-propynylphenethylamine (to be referred to as "salt of selegiline").

### BACKGROUND OF THE INVENTION

An antiparkinsonism drug, selegiline, is known as an inhibitor of monoamine oxidase (MAO), and it is also known that there are different subtypes of MAO, i.e., type A (MAO-A) and type B (MAO-B), and selegiline is a selective inhibitor of MAO-B. On the other hand, it is known that selegiline also inhibits MAO-A when it is orally administered in a large amount and shows anti-depression action. However, since a lot of MAO-A is present in the digestive organs, when MAO-A is inhibited by oral administration of selegiline, there is a possibility of causing sudden hypertension. Accordingly, an administration form of selegiline that has fewer possibility of transferring the drug to the digestive organs has been in demand.

It is considered that an adhesive preparation for administering a drug into the living body through the skin surface is suitable as an administration form in the case of administering selegiline at a large dose, since it is capable of avoiding absorption of a drug by digestive tracts and its first-pass effect at the liver. However, since a drug is mixed in the pressure-sensitive adhesive of the adhesive preparation, there is a problem of generating degradation products formed by an interaction and the like of various trace components with the drug. Therefore, in order to prevent formation of such degradation products, attempts have been made to reveal structures of the degradation products and add a degradation inhibitor (an antioxidant or a stabilizer).

For example, JP-A-11-79979 discloses that when 2-mercaptobenzimidazole and/or propyl gallate and a percutaneous absorption drug are contained in a pressure-sensitive adhesive layer containing an acrylic copolymer, 2-mercaptobenzimidazole and/or propyl gallate acts upon trace components which are present in the acrylic pressure-sensitive adhesive and cause a coloring phenomenon for example by their interaction with the percutaneous absorption drug, thereby showing an action to inhibit reaction of the percutaneous absorption drug with the trace components in the pressure-sensitive adhesive, so that the coloring phenomenon which occurs when the percutaneous absorption drug is blended in the pressure-sensitive adhesive or a coloring enhancing phenomenon during storage can be controlled, and the drug content in the preparation can also be stabilized.

However, examinations are not carried out on selegiline in JP-A-11-79979, so that the stabilizing effect when applied to selegiline is not clear.

EP 1 731 143 A1 discloses an adhesive preparation comprising a pressure-sensitive adhesive layer comprising selegiline HCl and sodium hydroxide in an amount of 1.00 mol or 0.99 mol equivalents based on 1 mol of the drug salt.

### SUMMARY OF THE INVENTION

Accordingly, a problem that the invention is to solve is to provide a selegiline-containing adhesive preparation which is markedly low in the reduction of the selegiline content during storage.

With the aim of solving the above-mentioned problem, the present inventors have conducted intensive studies and found that when selegiline is used as the drug and a metal hydroxide in a specified amount based on selegiline is incorporated together with an antioxidant, formation of impurities during storage of the preparation becomes markedly less, thereby resulting in the accomplishment of the invention.

Namely, the present invention provides the following items.
1. An adhesive preparation, which comprises a backing and a pressure-sensitive adhesive layer formed on at least one side of the backing, the pressure-sensitive adhesive layer comprising a pharmaceutically acceptable salt of (-)-(R)-_{N,α}-dimethyl-N-2-propynylphenethylamine, a pressure-sensitive adhesive, an antioxidant and a metal hydroxide, wherein the antioxidant is 2-mercaptobenzimidazole, and
   wherein the pressure-sensitive adhesive layer is prepared by, together with the pharmaceutically acceptable salt of (-)-(R)-N,α-dimethyl-N-2-propynylphenethylamine, incorporating the metal hydroxide in an amount of from 1.02 to 1.20 mol equivalents based on 1 mol of the salt.
2. The adhesive preparation according to item 1 , wherein the metal hydroxide is at least one compound selected from the group consisting of sodium hydroxide, calcium hydroxide and magnesium hydroxide.
3. The adhesive preparation according to any one of items 1 or 2, wherein
   the pressure-sensitive adhesive layer further comprises a liquid plasticizer.
4. The adhesive preparation according to any one of items 1 to 3, wherein
   the pressure-sensitive adhesive is an acrylic pressure-sensitive adhesive containing an acrylic polymer.

According to the invention, there can be provided an adhesive preparation which is high in stability of selegiline and is reduced in the amount of impurities formed.

### BRIEF DESCRIPTION OF THE DRAWING

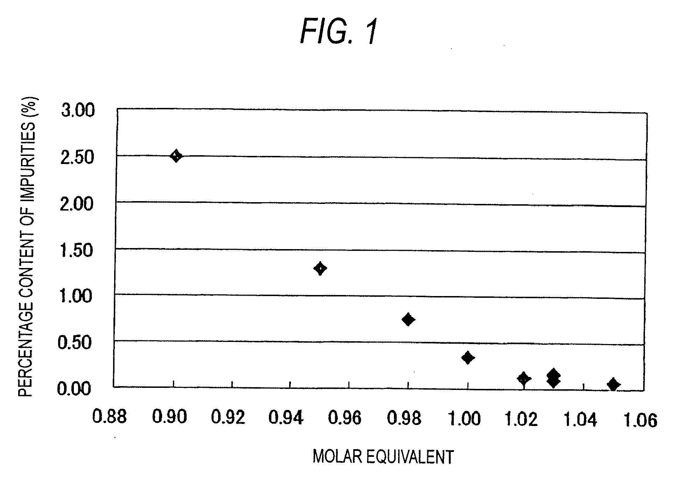

Fig. 1 shows a correlation of the molar equivalent of a metal hydroxide based on 1 mol of a selegiline salt with the percentage content of impurities.

### DETAILED DESCRIPTION OF THE INVENTION

### The following describes the invention in detail.

The adhesive preparation of the invention is for effecting percutaneous absorption of selegiline, contains selegiline in its pressure-sensitive adhesive layer and can be used as an antiparkinsonism drug and an antidepressant. In addition, as its other applications, there may be mentioned an anti-Alzheimer disease agent, an antiepileptic, seasickness prevention, treatment of schizophrenia, maintenance and protection of nerve cell function, improvement of acetylcholine system neurotransmitter, treatment of glaucoma, prevention of senescence, treatment of HIV-related cognition function disorder, treatment of ADHD (attention-deficit hyperactivity disorder) and the like.

The salt of selegiline as the active ingredient of the adhesive preparation of the invention can be contained in the pressure-sensitive adhesive layer in a dissolved state, a dispersed state and/or a crystalline state.

Accoording to the invention, when a salt of selegiline is contained in the pressure-sensitive adhesive layer, such an adhesive preparation is advantageous from the viewpoint that the stabilizing effect is strongly expressed.

As the salt of selegiline, for example, there may be mentioned a salt of an inorganic acid, such as hydrochloride, hydrobromide, phosphate, nitrate, sulfate and the like, and a salt of an organic acid, such as acetate, oxalate, maleate, fumarate, tartrate, succinate and the like. Of these salts, hydrochloride (to be referred also to as "selegiline hydrochloride" hereinafter) is preferable from the viewpoint that when neutralized with a metal hydroxide, a metal chloride such as sodium chloride and the like, which inhibits reduction of cohesive strength and cohesive failure of the pressure-sensitive adhesive layer and thereby contributes to the stabilization of the preparation, can be formed.

Content of the salt of selegiline in the pressure-sensitive adhesive layer is within the range of from 0.5% by weight to 30% by weight, preferably from 1% by weight to 20% by weight, based on the total weight of the pressure-sensitive adhesive layer. When the content thereof is smaller than 0.5% by weight, there is a possibility that the desired therapeutic and preventive effects cannot be obtained, while when it is larger than 30% by weight, there is a possibility that a side effect due to high concentration selegiline is expressed.

As the backing to be used in the invention, although there is no particular limitation, a material in which contents of a liquid plasticizer and selegiline are not reduced due to their loss from the backside through the backing, namely a material having impermeability for these components, is desirable. Illustratively, there may be mentioned a film made of a polyester such as polyethylene terephthalate, nylon, polyvinyl chloride, polyethylene, polypropylene, an ethylene-vinyl acetate copolymer, polytetrafluoroethylene, an ionomer resin and the like, a metal foil or a laminate film thereof and the like. Among them, in order to improve adhesiveness (anchoring property) with the pressure-sensitive adhesive layer, it is preferable to constitute the backing by a laminate film of a nonporous film made of the above-mentioned material with a porous film and form the pressure-sensitive adhesive layer on the porous film side.

The above-mentioned porous film is not particularly limited so long as the anchoring property of the pressure-sensitive adhesive layer is appropriate, and for example, there may be mentioned paper, woven fabric, non-woven fabric, a mechanically punching-treated sheet and the like, of which paper, woven fabric or non-woven fabric is particularly preferable. When improvement of the anchoring property and flexibility of the adhesive preparation are taken into consideration, thickness of such a porous film is generally from about 10 µm to about 500 µm, and in the case of a thin adhesive preparation such as a plaster type or pressure-sensitive adhesive tape type, it is generally from about 10 µm to about 200 µm. In addition, in the case of woven fabric and non-woven fabric, it is desirable to set their filling amount to a level of from 5 g/m² to 30 g/m² from the viewpoint of improving anchoring strength.

The pressure-sensitive adhesive layer according to the invention is formed on at least one side of the backing. As the pressure-sensitive adhesive to be contained in the pressure-sensitive adhesive layer of the invention, an acrylic pressure-sensitive adhesive, a rubber-based pressure-sensitive adhesive, a silicone-based pressure-sensitive adhesive, a vinyl ester-based pressure-sensitive adhesive and the like can be mentioned. Particularly, an acrylic pressure-sensitive adhesive containing an acrylic polymer is desirable from the viewpoint of skin adhesiveness as the adhesive preparation.

In general, the acrylic pressure-sensitive adhesive according to the invention is a polymer which comprises at least an alkyl ester of (meth)acrylic acid (to be also referred to as (meth)acrylic acid alkyl ester or alkyl (meth)acylate) as a monomer component, preferably a copolymer of an alkyl ester of (meth)acrylic acid with other monomer which is copolymerizable with the alkyl ester of (meth)acrylic acid (to be referred simply to as "other monomer" hereinafter), in which the main component is the alkyl ester of (meth)acrylic acid.

As the alkyl group of the (meth)acrylic acid alkyl ester, from the viewpoint of stickiness to the human skin, the number of carbon atoms is preferably 4 or more, particularly the number of carbon atoms is from 4 to 13, and it may be a straight chain or a branched chain. Illustratively, there may be mentioned butyl, isobutyl, sec-butyl, tert-butyl, pentyl, hexyl, heptyl, n-octyl, iso-octyl, sec-octyl, tert-octyl, 2-ethylhexyl, nonyl, decyl, undecyl, dodecyl, tridecyl and the like, of which 2-ethylhexyl of preferred. The (meth)acrylic acid alkyl ester can be used alone or by a combination of two or more species.

As the other monomer, examples thereof include carboxyl group-containing monomers such as (meth)acrylic acid, itaconic acid, maleic acid, maleic anhydride and the like; sulfoxyl group-containing monomers such as styrene sulfonate, allyl sulfonate, sulfopropyl (meth)acrylate, (meth)acryloyloxynaphthalene sulfonate, acrylamidomethyl sulfonate and the like; hydroxyl group-containing monomers such as hydroxyethyl (meth)acrylate, hydroxypropyl(meth)acrylate; (meth)acrylic acid derivatives having amido group such as (meth)acrylamide, dimethyl (meth)acrylamide, hydroxyethyl (meth)acrylamide, N-butyl (meth)acrylamide, N-methylol (meth)acrylamide and the like; aminoalkyl esters of (meth)acrylic acid such as aminoethyl (meth)acrylate, dimethylaminoethyl (meth)acrylate, tert-butylaminoethyl (meth)acrylate and the like; alkoxy esters of (meth)acrylic acid such as methoxyethyl (meth)acrylate, ethoxyethyl (meth)acrylate, tetrahydrofurfuryl (meth)acrylate and the like; alkoxyalkylene glycol esters of (meth)acrylic acid such as methoxyethylene glycol (meth)acrylate, methoxydiethylene glycol (meth)acrylate, methoxypolyethylene glycol (meth)acrylate, methoxypolypropylene glycol (meth)acrylate and the like; (meth)acrylonitrile; compounds having vinyl group such as vinyl acetate, vinyl propionate, N-vinyl-2-pyrrolidone, methylvinylpyrrolidone, vinylpyridine, vinylpiperidone, vinylpyrimidine, vinylpiperazine, vinylpyrrole, vinylimidazole, vinylcaprolactam, vinyloxazole, vinylmorpholine and the like, and these may be used alone or as a combination of two or more species. Particularly, carboxyl group-containing monomers (preferably acrylic acid), hydroxyl group-containing monomers (preferably 2-hydroxyethyl acrylate), (meth)acrylic acid derivatives having amido group (preferably hydroxyethyl (meth)acrylamide), N-vinyl-2-pyrrolidone, vinyl acetate and the like are desirable from the viewpoint of pressure-sensitive adhesive characteristics.

Copolymerization ratio of the alkyl ester of (meth)acrylic acid and other monomer is not particularly limited and is arbitrarily set in response to the molecular weight characteristics of the copolymer to be obtained, such as weight average molecular weight and the like. Particularly preferable is a copolymer obtained by blending the alkyl ester of (meth)acrylic acid and other monomer at a weight ratio of alkyl ester of (meth)acrylic acid/other monomer = generally 50 to 97/50 to 3, preferably 65 to 95/35 to 5, followed by copolymerization.

As a desirable copolymer, for example, there may be mentioned a copolymer of 2-ethylhexyl acrylate, N-vinyl-2-pyrrolidone and acrylic acid; a copolymer of 2-ethylhexyl acrylate, 2-hydroxyethyl acrylate and vinyl acetate; a copolymer of 2-ethylhexyl acrylate and acrylic acid, and the like. From the viewpoint of pressure-sensitive adhesive characteristics of the copolymer, more preferred is a copolymer of 2-ethylhexyl acrylate, N-vinyl-2-pyrrolidone and acrylic acid, and particularly preferred is a copolymer obtained by blending 2-ethylhexyl acrylate, N-vinyl-2-pyrrolidone and acrylic acid at a weight ratio of 2-ethylhexyl acrylate/N-vinyl-2-pyrrolidone/acrylic acid = 50 to 90/10 to 30/0 to 5, followed by copolymerization.

Content of the pressure-sensitive adhesive in the pressure-sensitive adhesive layer is within the range of from 20% by weight to 90% by weight, preferably from 30% by weight to 90% by weight, based on the total weight of the pressure-sensitive adhesive layer. When the content thereof is smaller than 20% by weight, there is a possibility that it becomes difficult to maintain the skin adhesive strength of the adhesive preparation.

A liquid plasticizer may be contained in the pressure-sensitive adhesive layer of the adhesive preparation of the invention. When a liquid plasticizer is contained in the pressure-sensitive adhesive layer, the pressure-sensitive adhesive layer is softened and thus skin irritation during wearing and/or at the time of peeling can be reduced. As such a liquid plasticizer, there is no particular limitation so long as the substance itself is liquid at 25°C, shows plasticizing action and is compatible with an adhesive polymer constituting the above-mentioned pressure-sensitive adhesive, and the substance which can improve percutaneous absorption property and storage stability of selegiline is desirable. In addition, a liquid plasticizer can also be blended for the purpose of further increasing solubility and the like of selegiline in the pressure-sensitive adhesive.

As such a liquid plasticizer, there may be mentioned a fatty acid ester containing a higher fatty acid having from 12 to 16 carbon atoms and a lower monovalent alcohol having from 1 to 4 carbon atoms (to be referred also to as "C12-16/C1-4 fatty acid ester" hereinafter); a fatty acid having 8 or 9 carbon atoms, such as caprylic acid (octanoic acid, C8), pelargonic acid (nonanoic acid, C9) and the like; a glycerol ester of middle chain fatty acid; glycols such as ethylene glycol, diethylene glycol, triethylene glycol, polyethylene glycol, propylene glycol, 1,3-propanediol, polypropylene glycol and the like; oils and fats such as olive oil, castor oil, squalene, lanolin and the like; an organic solvent such as ethyl acetate, ethyl alcohol, dimethyl sulfoxide, dimethylformamide, dimethylacetamide, dimethyllaurylamide, dodecylpyrrolidone, isosorbitol, oleyl alcohol, lauric acid, oleic acid, N-methyl-2-pyrrolidone and the like; a liquid surfactant; hydrocarbons such as liquid paraffin; a conventionally known plasticizer such as phthalic acid ester and the like; as well as ethoxylated stearyl alcohol, isotridecyl myristate, ethyl oleate, adipic acid diester, sebacic acid diester, octyl palmitate, glycerol and the like. These liquid plasticizers may be used as one species alone or by a combination of two or more species.

In the above-mentioned C12-16/C1-4 fatty acid ester, the higher fatty acid having from 12 to 16 carbon atoms includes saturated and unsaturated fatty acids but a saturated fatty acid is desirable, and the lower monovalent alcohol having from 1 to 4 carbon atoms may be a straight chain or a branched chain. As the suitable examples of the higher fatty acid having from 12 to 16 carbon atoms, lauric acid (C12), myristic acid (C14) and palmitic acid (C16) may be mentioned, and as the suitable examples of the lower monovalent alcohol having from 1 to 4 carbon atoms, isopropyl alcohol, ethyl alcohol, methyl alcohol, propyl alcohol and the like may be mentioned. As the suitable illustrative examples of the fatty acid ester, isopropyl myristate, ethyl laurate and isopropyl palmitate may be mentioned.

As the glycerol ester of middle chain fatty acid (middle chain fatty acid ester of glycerol), a glycerol ester of a fatty acid having from 8 to 12 carbon atoms is desirable, and it may be any one of monoglyceride, diglyceride and triglyceride. The fatty acid having from 8 to 12 carbon atoms includes saturated and unsaturated fatty acids but a saturated fatty acid is desirable, and for example, there may be mentioned caprylic acid (octanoic acid, C8), pelargonic acid (nonanoic acid, C9), capric acid (decanoic acid, C10) and the like. As the particularly desirable glycerol esters of middle chain fatty acid, a middle chain fatty acid diglyceride, a middle chain fatty acid triglyceride and the like may be mentioned, of which a middle chain fatty acid triglyceride is most desirable.

As the middle chain fatty acid triglyceride, preferred is a triglyceride in which at least one of the three fatty acids bonding to glycerol by an ester bond is a middle chain fatty acid (the number of carbons therein is from 8 to 12), more preferred is a triglyceride in which at least two of the three fatty acids bonding to glycerol by an ester bond are a middle chain fatty acid (the number of carbons therein is from 8 to 12), and most preferred is a triglyceride in which all of the three fatty acids bonding to glycerol by an ester bond are a middle chain fatty acid (the number of carbons therein is from 8 to 12).

Also, in the middle chain fatty acid triglyceride, a triglyceride in which the middle chain fatty acid species (in which the number of carbons is from 8 to 12) that bonds to glycerol by an ester bond is only one species (e.g., caprylic acid triglyceride in which the middle chain fatty acid bonding to glycerol by an ester bond is caprylic acid alone, capric acid triglyceride in which the middle chain fatty acid bonding to glycerol by an ester bond is capric acid alone, and the like) may be used, or a triglyceride in which the middle chain fatty acid species (in which the number of carbons is from 8 to 12) that bonds to glycerol by an ester bond are two or more species (e.g., (caprylic acid/capric acid) triglyceride in which the middle chain fatty acids that bond to glycerol by an ester bond are caprylic acid and capric acid, (caprylic acid/capric acid/lauric acid) triglyceride in which the middle chain fatty acids that bond to glycerol by an ester bond are caprylic acid, capric acid and lauric acid, and the like) may be used. As the middle chain fatty acid triglyceride in the invention, one species of middle chain fatty acid triglyceride alone may be used or a mixture of two or more species of middle chain fatty acid triglyceride may be used.

In addition, the middle chain fatty acid triglyceride may be an extract from a natural material or a synthesized product. In addition, a commercial item can also be used, and for example, there may be mentioned "COCONARD" manufactured by Kao Corp., "Crodamol GTCC" manufactured by Croda Inc., "PANACET 810S" manufactured by NOF CORPORATION and the like.

As the middle chain fatty acid diglyceride (in which the number of carbons is from 8 to 12), for example, caprylic acid diglyceride in which the middle chain fatty acid is caprylic acid alone may be mentioned. The middle chain fatty acid diglyceride may be an extract from a natural material or a synthesized product. In addition, a commercial item can also be used.

Preferred as the adipic acid diester is a diester in which the number of carbons of the alcohol residue that bonds to adipic acid by a ester bond is from 1 to 4, and for example, there may be mentioned dimethyl adipate, diethyl adipate, diisopropyl adipate, dibutyl adipate and the like, of which diisopropyl adipate is particularly desirable.

Preferred as the sebacic acid diester is a diester in which the number of carbons of the alcohol residue that bonds to sebacic acid by a ester bond is from 1 to 5, and for example, there may be mentioned dimethyl sebacate, diethyl sebacate, diisopropyl sebacate and the like, of which diisopropyl sebacate is particularly desirable.

According to the invention, from the viewpoint of compatibility with a pressure-sensitive adhesive (particularly an acrylic pressure-sensitive adhesive), storage stability of selegiline and the like, the liquid plasticizer is preferably a C12-16/C1-4 fatty acid ester, a fatty acid having 8 or 9 carbon atoms, a middle chain fatty acid glycerol ester or an adipic acid diester, more preferably a C12-16/C1-4 fatty acid ester, a middle chain fatty acid glycerol ester or an adipic acid diester, particularly preferably isopropyl myristate, a middle chain fatty acid triglyceride (e.g., (caprylic acid/capric acid) triglyceride) or diisopropyl adipate.

Content of the liquid plasticizer when the pressure-sensitive adhesive layer contains the liquid plasticizer is within the range of, for example, from 2% by weight to 60% by weight, preferably from 20% by weight to 50% by weight, more preferably from 30% by weight to 50% by weight, based on the total weight of the pressure-sensitive adhesive layer. When the content thereof is less than 2% by weight, there may be a case in which the skin irritation cannot be reduced due to insufficient plasticization of the pressure-sensitive adhesive layer. When it exceeds 60% by weight on the contrary, there may be a case in which the liquid plasticizer cannot be kept in the pressure-sensitive adhesive even by the cohesive force possessed by the pressure-sensitive adhesive, and there may be a case in which the adhesiveness becomes poor due to the blooming of the liquid plasticizer on the surface of the pressure-sensitive adhesive layer. In addition, by crosslinking a pressure-sensitive adhesive layer containing 20% by weight or more of the liquid plasticizer, it becomes possible to provide an adhesive preparation which has softness and shows low skin irritation at the time of peeling.

According to the adhesive preparation of the invention, the pressure-sensitive adhesive layer may be non-crosslinked, but in the case of preventing excess plasticization, a crosslinking treatment may be applied to the pressure-sensitive adhesive layer. In that case, as the crosslinking agent for applying a crosslinking treatment to the pressure-sensitive adhesive layer, there is no particular limitation so long as the crosslink formation is not inhibited by selegiline, and for example, there may be mentioned an organic metal compound (e.g., zirconium, zinc, zinc acetate and the like), a metal alcoholate (e.g., tetraethyl titanate, tetraisopropyl titanate, aluminum isopropylate, aluminum sec-butylate and the like) and a metal chelate compound (e.g., dipropoxybis(acetylacetonate) titanium, tetraoctylene glycol titanium, aluminum isopropylate, ethyl acetoacetate aluminum diisopropylate, aluminum tris(ethyl acetoacetate), aluminum tris(acetyl acetonate) and the like), of which a metal chelate compound is preferable. Particularly, ethyl acetoacetate aluminum diisopropylate is more preferable. In the crosslinking treatment, the above-mentioned crosslinking agents may be used alone or as a combination of two or more species.

When the crosslinking treatment is applied to the pressure-sensitive adhesive layer, content of the crosslinking agent varies depending on the kinds of the crosslinking agent and pressure-sensitive adhesive, but is generally from 0.05% by weight to 0.6% by weight based on the total weight of the pressure-sensitive adhesive layer.

The adhesive preparation of the invention contains an antioxidant in the pressure-sensitive adhesive layer. It is considered that formation of impurities can be controlled by the inclusion of the antioxidant, because the reaction of the salt of selegiline with trace components in the pressure-sensitive adhesive layer is inhibited. As such an antioxidant, 2-mercaptobenzimidazole is used.

Content of the antioxidant varies depending on the kinds of the antioxidant and pressure-sensitive adhesive, but since there is a possibility that skin irritation due to the antioxidant occurs when blended in a too large amount, it is generally 5.0% by weight or less, preferably 2.0% by weight or less, based on the total weight of the pressure-sensitive adhesive layer.

The adhesive preparation of the invention contains a metal hydroxide in the pressure-sensitive adhesive layer. Owing to the inclusion of a metal hydroxide, stability of the drug in the preparation is improved. As the metal hydroxide, for example, sodium hydroxide, calcium hydroxide, magnesium hydroxide and the like may be mentioned, of which sodium hydroxide is preferable.

Amount of the metal hydroxide to be incorporated is of from 1.02 to 1.20 mol equivalents based on 1 mol of the salt of selegiline. When the incorporating amount of the metal hydroxide is larger than 1.02 mol equivalent, formation of certain kinds of impurities can be sufficiently controlled. Also, when incorporated in a too large amount, there is a possibility of generating skin irritation due to too much increase of pH of the adhesive preparation and also there is a possibility of reducing production efficiency due to increase of viscosity of a composition for pressure-sensitive adhesive layer formation during the production thereof. Therefore, the amount is 1.20 equivalents or less, preferably 1.10 equivalent or less, based on 1 mol of the salt of selegiline. In this connection, when the upper limit of the incorporation amount of the metal hydroxide exceeds 1.20 equivalents, there is a possibility of exerting influence upon the crosslinking reaction when the pressure-sensitive adhesive layer is subjected to a crosslinking treatment. The "certain kinds of impurities" according to the invention mean those which are specifically formed when selegiline is contained and show a peak at around a retention time of from 38 to 39 minutes by a high performance liquid chromatography (HPLC) analysis carried out under the following conditions.

**Table 1**

| | |
|---|---|
| Apparatus | Prominence, mfd. by Shimadzu Corp. |
| Detector | Absorptiometer (measuring wavelength: 205 nm) |
| Column | Kaseisorb LC ODS 2000 (particle diameter 5 µm, 4.6 mm ID x 250 mm), mfd. by Tokyo Chemical Industry |
| Column temp. | 25°C |
| Flow rate | 0.9 ml/min |
| Mobile phase | Mobile phase A/mobile phase B is made to flow through at the ratios show in the following Table 2. |
| Injection volume | 20 µl |
| Assay time | 60 minutes |

**Table 2**

| Time after injection (min) | Mobile phase A (% by volume) | Mobile phase B (% by volume) |
|---|---|---|
| 0 to 15 | 100 | 0 |
| 15 to 40 | 100 → 50 | 0 → 50 |
| 40 to 40.01 | 50 → 100 | 50 → 0 |
| 40.01 to 60 | 100 | 0 |

The mobile phase A is composed of ammonium dihydrogenphosphate solution (pH 3.1), acetonitrile and methanol at a ratio of 16/3/1, and the mobile phase B is composed of ammonium dihydrogenphosphate solution (pH 3.1), acetonitrile and methanol at a ratio of 6/13/1.

When the free form of selegiline is used, incorporation amount of the metal hydroxide is a result of subtracting one mol equivalent from the aforementioned incorporation amount in the case of using a salt of selegiline.

From the viewpoint of applying to and releasing from the skin surface, thickness of the pressure-sensitive adhesive layer is generally from 10 µm to 300 µm, preferably from 50 µm to 200 µm.

According to the necessity, the pressure-sensitive adhesive layer can be blended with additive agents such as various pigments, various fillers, a stabilizer, drug solubilizing agents, drug solubilization inhibitors and the like.

From the viewpoint of adhesion to skin, the pressure-sensitive adhesive layer is preferably a hydrophobic pressure-sensitive adhesive layer and more preferably a non-hydroscopic pressure-sensitive adhesive layer. The term "non-hydroscopic pressure-sensitive adhesive layer" as used herein is not always limited to those which are completely free from moisture, but those which contain a slight amount of moisture derived from the air humidity, the skin and the like are included therein. The term "a slight amount of moisture" as used herein is, as the moisture content of the layered product of backing and pressure-sensitive adhesive layer, preferably 5% by weight or less, more preferably 2% by weight or less, most preferably 1% by weight or less. In this case, the moisture content of the layered product of backing and pressure-sensitive adhesive layer means weight ratio of water contained in the layered product of backing and pressure-sensitive adhesive layer after separating a release liner when present (i.e., weight percentage of water based on the total weight of the layered product of backing and pressure-sensitive adhesive layer) which is measured by the coulometric Karl Fischer titration method, and is illustratively as follows. That is, under an environment controlled at a temperature of 23 ± 2°C and a relative humidity of 40 ± 5% RH, a test piece is prepared by punching a sample having a release liner when present, into a predetermined size. Then, after peeling off the release liner when present, the resulting test piece is put into a moisture vaporizer. The test piece is heated at 140°C in the moisture vaporizer, the moisture generated is then introduced into a titration flask using nitrogen as the carrier, and the moisture content (% by weight) of the sample is measured by the coulometric Karl Fischer titration method.

The production method of the adhesive preparation of the invention is not particularly limited, but for example, it can be produced by the following production method.

A drug-containing solution is prepared by mixing and stirring a salt of selegiline together with the above-mentioned metal hydroxide and the like in a solvent, thereby neutralizing the mixture.

The above-mentioned drug-containing solution is dissolved or dispersed in a solvent or dispersion medium together with, for example, a pressure-sensitive adhesive (e.g., an acrylic pressure-sensitive adhesive and the like), an antioxidant and, in response to the necessity, a crosslinking agent, a liquid plasticizer and other additive agents and the like. In this connection, the salt of selegiline has a tendency to become a dispersed state due to its low solubility in the pressure-sensitive adhesive layer. The solvent or dispersion medium to be used in forming the pressure-sensitive adhesive layer is not particularly limited, and those which are generally used as a solvent and the like of a pressure-sensitive adhesive can be selected by taking kind of the pressure-sensitive adhesive, its reactivity with the drug, and the like into consideration. As such a solvent or dispersion medium, ethyl acetate, toluene, hexane, 2-propanol, methanol, ethanol and the like may for example be mentioned.

Next, a pressure-sensitive adhesive layer is formed by coating the thus obtained solution or dispersion on one side of the backing or the release treatment side of a release sheet, followed by drying. In this connection, it is possible to carry out the aforementioned coating by, for example, a technique conventionally known to those skilled in the art, such as casting, printing and the like. Thereafter, the release sheet or backing is pasted to the pressure-sensitive adhesive layer. As such a release sheet, there is no particular limitation so long as it can be easily peeled off from the pressure-sensitive adhesive layer when used, and for example, there may be used a film such as of polyester, polyvinyl chloride, polyvinylidene chloride, polyethylene terephthalate and the like in which a silicone treatment was applied to its contacting side with the pressure-sensitive adhesive layer, or a laminated film of wood free paper or glassine paper with polyolefin, and the like. Thickness of the release sheet is generally 200 µm or less, preferably from 25µm to 100 µm. The adhesive preparation of the invention is prepared by, after pasting the release sheet to the pressure-sensitive adhesive layer, accelerating the crosslinking reaction by applying an aging treatment and the like at generally from 60°C to 90°C, preferably from 60°C to 70°C, for a period of from 24 hours to 48 hours.

Shape of the adhesive preparation of the invention is not limited, and for example, it may be a tape shape, a sheet shape and the like.

Dose of the adhesive preparation of the invention varies depending on the age, body weight, symptoms and the like of each patient, but it is desirable to apply an adhesive preparation containing from 1 mg to 40 mg of selegiline, generally to the skin of an adult within an area of from 1 cm² to 40 cm² approximately from once per two days to twice a day.

### Examples

The following describes the invention in detail with reference to Examples and Comparative Examples, though these Examples and Comparative Examples do not limit the invention. In this connection, the "part(s)" and "%" as used in the following mean "part(s) by weight" and "% by weight", respectively, unless otherwise noted.

### (Preparation of acrylic pressure-sensitive adhesive)

Under an inert gas atmosphere, 72 parts of 2-ethylhexyl acrylate (2-EHA), 25 parts of N-vinyl-2-pyrrolidone, 3 parts of acrylic acid and 0.2 part of azobisisobutyronitrile were allowed to undergo solution polymerization in ethyl acetate at 60°C, thereby preparing a solution of an acrylic pressure-sensitive adhesive.

### (Preparation of selegiline-containing adhesive preparations of Examples 1 to 6 and Comparative Examples 1 to 4)

Each pressure-sensitive adhesive solution was prepared in accordance with the blending ratio shown in the following Table 3, its viscosity was adjusted with isopropanol, and the thus obtained solution was coated on a polyester film (75 µm in thickness) so that the thickness of a pressure-sensitive adhesive layer after drying became 80 µm and then dried to prepare the pressure-sensitive adhesive layer. Subsequently, this pressure-sensitive adhesive layer was pasted on a polyester film (12 µm in thickness) and then an aging treatment was carried out at 60°C for 48 hours, thereby preparing a selegiline-containing adhesive preparation.

Molar equivalents of the metal hydroxide based on 1 mol selegiline salt, in the adhesive preparations of respective Examples and Comparative Examples, are shown in Table 4. In this connection, "COCONARD MT" ((caprylic acid/capric acid) triglyceride, mfd. by Kao Corp.) was used as the middle chain fatty acid triglyceride.

In addition, the "ALCH" in Table 3 represents ethyl acetoacetate aluminum diisopropylate.

**Table 3**

| | Acrylic copolymer | Liquid plasticizer | | ALCH | 2-Mercapto benzimidazole | Selegiline hydrochloride | NaOH |
|---|---|---|---|---|---|---|---|
| | | Name | Content | | | | |
| Example 1 | 46.33 | Isopropyl myristate | 39.40 | 0.14 | 0.30 | 11.70 | 2.13 |
| Example 2 | 46.33 | Isopropyl myristate | 39.34 | 0.14 | 0.30 | 11.70 | 2.19 |
| Example 3 | 84.69 | Isopropyl myristate | 2.00 | - | 0.30 | 11.00 | 2.01 |
| Example 4 | 87.05 | Isopropyl myristate | 2.00 | - | 0.30 | 9.00 | 1.65 |
| Example 5 | 50.70 | Diisopropyl adipate | 35.00 | 0.15 | 0.30 | 11.70 | 2.15 |
| Example 6 | 50.72 | Medium chain fatty acid triglyceride | 35.00 | 0.13 | 0.30 | 11.70 | 2.15 |
| Comparative Example 1 | 46.33 | Isopropyl myristate | 39.65 | 0.14 | 0.30 | 11.70 | 1.88 |
| Comparative Example 2 | 46.33 | Isopropyl myristate | 39.54 | 0.14 | 0.30 | 11.70 | 1.99 |
| Comparative Example 3 | 46.33 | Isopropyl myristate | 39.48 | 0.14 | 0.30 | 11.70 | 2.05 |
| Comparative Example 4 | 46.33 | Isopropyl myristate | 39.44 | 0.14 | 0.30 | 11.70 | 2.09 |

| | | | | | | | |
|---|---|---|---|---|---|---|---|
| Unit: % by weight based on total weight of pressure-sensitive adhesive layer | | | | | | | |

**Table 4**

| | Molar equivalent |
|---|---|
| Example 1 | 1.02 |
| Example 2 | 1.05 |
| Example 3 | 1.03 |
| Example 4 | 1.03 |
| Example 5 | 1.03 |
| Example 6 | 1.03 |
| Comparative Example 1 | 0.90 |
| Comparative Example 2 | 0.95 |
| Comparative Example 3 | 0.98 |
| Comparative Example 4 | 1.00 |

### (Measurement of percentage content of impurities)

Each of the selegiline-containing adhesive preparations of Examples 1 to 6 and Comparative Examples 1 to 4 was stored for 3 months under a temperature condition of 50 ± 2°C. Thereafter, measurement of the percentage content of impurities was carried out by the method shown below.

Each of the adhesive preparations was stamped out into an appropriate size and extracted with an organic solvent on a shaker, and the extracted solution was measured using a HPLC.

Assay conditions of the HPLC are shown in the following Table 5 and Table 6.

**Table 5**

| | |
|---|---|
| Apparatus | Prominence, mfd. by Shimadzu Corp. |
| Detector | Absorptiometer (measuring wavelength: 205 nm) |
| Column | Kaseisorb LC ODS 2000 (particle diameter 5 µm, 4.6 mm ID x 250 mm), mfd. by Tokyo Chemical Industry |
| Column temp. | 25°C |
| Flow rate | 0.9 ml/min |
| Mobile phase | Mobile phase A/mobile phase B was made to flow through at the ratios of the following Table 6. |
| Injection volume | 20 µl |
| Assay time | 60 minutes |

**Table 6**

| Time after injection (min) | Mobile phase A (% by volume) | Mobile phase B (% by volume) |
|---|---|---|
| 0 to 15 | 100 | 0 |
| 15 to 40 | 100 → 50 | 0 → 50 |
| 40 to 40.01 | 50 → 100 | 50 → 0 |
| 40.01 to 60 | 100 | 0 |

In this connection, a mixture composed of ammonium dihydrogenphosphate solution (pH 3.1), acetonitrile and methanol at a ratio of 16/3/1 was used as the mobile phase A, and a mixture composed of ammonium dihydrogenphosphate solution (pH 3.1), acetonitrile and methanol at a ratio of 6/13/1 was used as the mobile phase B, respectively.

A peak area of certain impurities (a peak area of at around 38 minutes in retention time) was divided by the peak area of the main drug, and the result was multiplied by 100 and used as the percentage content of impurities. In this connection, the tests were carried out by the number of tests of n = 3.

The results are shown in Table 7.

**Table 7**

| | Percentage content of impurities (%) |
|---|---|
| Example 1 | 0.13 |
| Example 2 | 0.07 |
| Example 3 | 0.17 |
| Example 4 | 0.17 |
| Example 5 | 0.10 |
| Example 6 | 0.15 |
| Comparative Example 1 | 2.50 |
| Comparative Example 2 | 1.30 |
| Comparative Example 3 | 0.76 |
| Comparative Example 4 | 0.35 |

According to Examples 1 to 6, adhesive preparations having low percentage content of impurities were obtained. On the other hand, it was confirmed that the percentage content of impurities is high in the adhesive preparations of Comparative Examples 1 to 4. Accordingly, it was not able to obtain an adhesive preparation having low percentage content of impurities by the comparative examples.

This application is based on Japanese patent application No. 2010-056629 filed March 12, 2010.

## Claims

1. An adhesive preparation, which comprises a backing and a pressure-sensitive adhesive layer formed on at least one side of the backing, the pressure-sensitive adhesive layer comprising a pharmaceutically acceptable salt of (-)-(R)-N,α-dimethyl-N-2-propynylphenethylamine, a pressure-sensitive adhesive, an antioxidant and a metal hydroxide,
wherein the antioxidant is 2-mercaptobenzimidazole; and
wherein the pressure-sensitive adhesive layer is prepared by, together with the pharmaceutically acceptable salt of (-)-(R)-N,α-dimethyl-N-2-propynylphenethylamine, incorporating the metal hydroxide in an amount of from 1.02 to 1.20 mol equivalents based on 1 mol of the salt.

2. The adhesive preparation of claim 1, wherein the metal hydroxide is at least one compound selected from the group consisting of sodium hydroxide, calcium hydroxide and magnesium hydroxide.

3. The adhesive preparation of claim 1 or 2, wherein the pressure-sensitive adhesive layer further comprises a liquid plasticizer.

4. The adhesive preparation of any one of claims 1 to 3, wherein the pressure-sensitive adhesive is an acrylic pressure-sensitive adhesive containing an acrylic polymer.

## Patentansprüche

1. Haftmittelzubereitung, welche einen Träger und eine Haftklebstoffschicht, die auf wenigstens einer Seite des Trägers gebildet ist, umfasst, wobei die Haftklebstoffschicht ein pharmazeutisch verträgliches Salz von (-)-(R)-N,α-Dimethyl-N-2-propinylphenethylamin, einen Haftklebstoff, ein Antioxidationsmittel und ein Metallhydroxid umfasst,
wobei das Antioxidationsmittel 2-Mercaptobenzimidazol ist; und
wobei die Haftklebstoffschicht dadurch hergestellt wird, dass, zusammen mit dem pharmazeutisch verträglichen Salz von (-)-(R)-N,α-Dimethyl-N-2-propinylphenethylamin, das Metallhydroxid in einer Menge von 1,02 bis 1,20 Moläquivalenten, bezogen auf 1 Mol des Salzes, eingearbeitet wird.

2. Haftmittelzubereitung nach Anspruch 1, wobei das Metallhydroxid wenigstens eine Verbindung, ausgewählt aus der Gruppe bestehend aus Natriumhydroxid, Calciumhydroxid und Magnesiumhydroxid, ist.

3. Haftmittelzubereitung nach Anspruch 1 oder 2, wobei die Haftklebstoffschicht außerdem einen flüssigen Weichmacher umfasst.

4. Haftmittelzubereitung nach einem der Ansprüche 1 bis 3, wobei der Haftklebstoff ein Acrylhaftklebstoff ist, der ein Acrylpolymer enthält.

## Revendications

1. Préparation adhésive, laquelle comprend un support et une couche adhésive à la pression formée sur au moins un côté du support, la couche adhésive sensible à la pression comprenant un sel pharmaceutiquement acceptable de (-)-(R)-N,α-diméthyl-N-2-propynyl-phénéthylamine, un adhésif sensible à la pression, un antioxydant et un hydroxyde de métal,
dans laquelle l'antioxydant est le 2-mercaptobenzimidazole ; et
dans laquelle la couche adhésive sensible à la pression est préparée par, avec le sel pharmaceutiquement acceptable de (-)-(R)-N,α-diméthyl-N-2-propynylphénéthylamine, l'incorporation de l'hydroxyde de métal dans une quantité de 1,02 à 1,20 équivalents mole rapporté à une mole du sel.

2. Préparation adhésive selon la revendication 1, dans laquelle l'hydroxyde de métal est au moins un composé choisi dans le groupe constitué d'hydroxyde de sodium, d'hydroxyde de calcium et d'hydroxyde de magnésium.

3. Préparation adhésive selon la revendication 1 ou 2, dans laquelle la couche adhésive sensible à la pression comprend de plus un plastifiant liquide.

4. Préparation adhésive selon l'une quelconque des revendications 1 à 3, dans laquelle l'adhésif sensible à la pression est un adhésif sensible à la pression acrylique contenant un polymère acrylique.
